# FASCICULE DE BREVET EUROPEEN

(11) **EP 0 915 814 B1**
(45) Date de publication et mention de la délivrance du brevet: **14.11.2001**
(21) Numéro de dépôt: 97935657.3
(22) Date de dépôt: 30.07.1997
(51) Int. Cl.: C07B 37/00, C07B 45/00, C07C 319/14, C07C 321/28, C07C 313/04, C07C 313/02

(54) **PROCEDE POUR GREFFER UN GROUPEMENT DIFLUOROMETHYLE SUBSTITUE**
VERFAHREN ZUM ANBRINGEN EINER SUBSTITUIERTEN DIFLUORMETHYLGRUPPE
METHOD FOR GRAFTING A SUBSTITUTED DIFLUOROMETHYLE GROUP

(30) Priorité: 01.08.1996 FR 9609753; 01.08.1996 FR 9609754
(43) Date de publication de la demande: 19.05.1999
(73) Titulaire: RHONE-POULENC CHIMIE, 92408 Courbevoie Cédex (FR)
(72) Inventeur: FORAT, Gérard, F-69003 Lyon (FR); MAS, Jean-Manuel, F-69330 Millery (FR); SAINT-JALMES, Laurent, F-69330 Meyzieu (FR)
(74) Mandataire: Jacobson, Claude
(86) Numéro de dépôt international: FR9701423
(87) Numéro de publication internationale: WO9805609

(56) Documents cités:
- EP-A- 0 165 135
- EP-A- 0 700 885
- FR-A- 2 593 808
- FR-A- 2 660 923
- G.P. STAHLY: "Trifluoromethylation of 1,3,5-trinitrobenzene" JOURNAL OF FLUORINE CHEMISTRY, vol. 45, no. 3, décembre 1989, LAUSANNE, CH, pages 431-433, XP002029697
- K. MATSUI, ET AL.: "A convenient trifluoromethylation of aromatic halides with sodium trifluoroacetate" CHEMISTRY LETTERS, no. 12, décembre 1981, TOKYO, JP, pages 1719-1720, XP002029698
- G.E. CARR, ET AL.: "Sodium perfluoroalkane carboxylates as sources of perfluoroalkyl groups" JOURNAL OF THE CHEMICAL SOCIETY, PERKIN TRANSACTIONS 1, no. 4, avril 1988, LETCHWORTH, GB, pages 921-926, XP002029699

## Description

La présente invention concerne un procédé pour greffer un groupement difluorométhyle substitué sur un composé comportant au moins une fonction électrophile et notamment utile pour préparer des acides difluoro- ou trifluorométhane sulfinique et sulfonique et leurs sels.

Elle concerne plus particulièrement une technique pour perfluoroalcoyler différents composés par des réactions de substitution nucléophile ou d'addition typiquement réalisées par des dérivés organométalliques.

Les techniques de perfluoroalcoylation, ou les techniques équivalentes, utilisent en général des dérivés du type iodure de perfluoroalcoyle, en présence de zinc. Cette technique est donc coûteuse, tout en nécessitant des installations de traitement des rejets métalliques qu'il convient de traiter car le zinc est un polluant important des cours d'eau.

Les autres techniques, où le radical perfluoroalcoyle ne forme pas un intermédiaire réactif stabilisé du type organo-métallique, sont en général difficiles à mettre en oeuvre en raison de la très faible stabilité des anions perfluorés libres dans les milieux réactionnels. Ces derniers conduisent en général à des produits du type carbène, lesquels lorsqu'ils réagissent ont perdu un de leurs substituants.

Dans le cas particulier des acides perhalogénoalcane sulfoniques et plus particulièrement de l'acide trifluorométhane sulfonique, utilisés comme catalyseurs ou comme intermédiaires en synthèse organique, le procédé initialement connu pour préparer l'acide trifluorométhane sulfonique, était la fluoration électrochimique telle que décrite notamment par R. D. Howels, J. D. Mc Cown dans Chemical Reviews, 1977, 77, 69.

A ce jour, on connaît également le procédé de préparation d'acide trifluorométhanesulfinique, décrit dans le brevet européen publié sous le numéro EP-165 135. Il consiste à mettre en présence de bioxyde de soufre un métal choisi parmi le zinc, l'aluminium, le manganèse, le cadmium, le magnésium, l'étain et le fer, voire le nickel et le cobalt, dans un solvant aprotique polaire puis à ajouter un halogénure de trifluorométhyle sous une pression supérieure à 10⁵ Pa. Ce procédé permet d'obtenir un produit sous forme de trifluorométhane sulfinate avec de bons rendements. Toutefois, le sulfinate obtenu se trouve dans un milieu contenant une quantité importante de sel de zinc. La séparation du sulfinate et des autres sels de zinc pose au niveau industriel un problème à résoudre. Par ailleurs, cette technique, ainsi que celle décrite dans la demande française publiée sous le numéro 2 593 808, nécessitaient l'utilisation de bromures de perfluoroalcoyles qui sont réputés particulièrement nocifs pour les couches atmosphériques, notamment en raison de leur fort effet de serre et de leur effet réputé néfaste sur l'ozone.

C'est pourquoi un des buts de la présente invention est de fournir un réactif qui permette une perfluoroalcoylation selon un mécanisme du type faisant intervenir carbanion, sans faire appel à des organométalliques de métaux de transition comme le zinc et qui utilise des produits moins nocifs pour l'environnement, que par exemple le bromure de trifluorométhyle tout en restant de prix peu élevé.

On a souvent cherché à utiliser comme source de radicaux perfluoroalcoyle, plus généralement de radicaux trifluorométhyle, des acides perfluorocarboxyliques, en mettant en oeuvre des réactions de décomposition visant à éliminer le fragment carboxylique desdits acides en libérant du bioxyde de carbone. Toutefois, les succès qui avaient été obtenus étaient très mitigés et utilisaient des systèmes catalytiques particulièrement compliqués. Les radicaux perfluoroalcoyle ou leurs équivalents engendrés par la décomposition desdits acides perfluorocarboxyliques étaient en outre instables dans le milieu réactionnel et nécessitaient l'emploi d'agents stabilisants.

La présente invention se propose d'obvier aux inconvénients des procédés existants en fournissant un réactif non nocif pour l'environnement et capable de conduire aux produits désirés avec un rendement satisfaisant.

Au cours de l'étude qui a mené à la présente invention, il a été démontré qu'une réaction de fluoroalcoylation était possible avec un sel d'acide fluorocarboxylique, sans catalyseur et sans agent susceptible de stabiliser les divers intermédiaires envisagés obtenus lors de la décomposition des différents acides perfluorocarboxyliques, en travaillant
* Matsui et al (Chemistry Letters, n° 12, 1981, pp 1719-20) décrit un procédé de trifluorométhylation d'halogénures aromatiques à l'aide du trifluoroacétate de sodium en présence d'iodure de cuivre. La réaction est effectuée dans la N-méthylpyrrolidone en tant que solvant.

EP-A-700 885 décrit un procédé de perfluoroalkylation et/ou perfluoracyloxylation de composés organiques consistant à mettre le substrat en contact avec un anhydride perfluoroalcanoïque en présence d'un peroxyde inorganique à une température supérieure à 10°C.

FR-A-2 660 923 décrit la réaction d'oxyde de soufre avec un perhalogénoalcane.

EP-A-733 614 décrit un réactif et un procédé utiles pour greffer un groupement difluorométhyle substitué sur un composé comportant au moins une fonction électrophile. Plus précisément, le procédé consiste à mettre en présence un acide fluorocarboxylique comportant un groupe électroattracteur dans un solvant aprotique polaire avec un composé comportant au moins une fonction électrophile, et à chauffer le mélange résultant à une température comprise entre 100 et 200°C pendant une durée comprise entre une demi-heure et un jour.

EP-A-753 023 décrit également un réactif et un procédé pour la synthèse de dérivés organiques oxysulfurés et fluorés. Plus précisément, le procédé consiste à mettre en présence un acide fluorocarboxylique du même type que précédemment avec un oxyde de soufre et à chauffer le mélange résultant à une température comprise entre 100° et 200°C pendant une durée comprise entre une demi-heure et vingt heures. dans un solvant aprotique polaire et sous l'action de micro-ondes.

Les buts précités et d'autres, qui apparaîtront par la suite, sont donc atteints selon l'invention par un procédé pour greffer un groupement difluorométhyle substitué sur un composé comportant au moins une fonction électrophile, qui comprend les étapes consistant à :
i) mettre ledit composé comportant au moins une fonction électrophile en présence d'un réactif nucléophile comprenant :
   a) un acide fluorocarboxylique de formule Ea - CF₂ - COOH où Ea représente un atome ou un groupe électroattracteur, au moins partiellement salifié par un cation organique ou minéral, et
   b) un solvant aprotique polaire ; puis
ii) exposer le milieu réactionnel à l'action de micro-ondes.

Les fonctions électrophiles susceptibles de réagir avec le réactif de la présente invention sont les fonctions qui réagissent habituellement avec les organométalliques et seront détaillées par la suite.

Le premier élément essentiel de l'invention concerne le réactif.

Ainsi qu'on l'a mentionné ci-dessus, le solvant joue un rôle important dans la présente invention et doit être aprotique, et avantageusement polaire et comporte de préférence très peu d'impuretés porteuses d'hydrogène acide, comme on le verra par la suite.

Il est ainsi préférable que le solvant aprotique polaire utilisable ait un moment dipolaire significatif. Ainsi, sa constante diélectrique relative e est avantageusement au moins égale à environ 5 (les zéros de position ne sont pas considérés comme des chiffres significatifs dans la présente description à moins qu'il en soit précisé autrement). De préférence l'e est inférieur ou égal à 50 et supérieur ou égal à 5.

On préfère en outre que les solvants de l'invention soient susceptibles de bien solvater les cations, ce qui peut être codifié par l'indice donneur D de ces solvants. Il est ainsi préférable que l'indice donneur D de ces solvants soit compris entre 10 et 30. Ledit indice donneur correspond au DH (variation d'enthalpie), exprimé en kilo calorie, de l'association dudit solvant aprotique polaire avec le pentachlorure d'antimoine.

Selon la présente invention, il est préférable que le réactif ne présente pas d'hydrogène acide sur le ou les solvants polaires qu'il utilise. En particulier lorsque le caractère polaire du ou des solvants est obtenu par la présence de groupes électroattracteurs, il est souhaitable qu'il n'y ait pas d'hydrogène en alpha de la fonction électroattractrice.

De manière plus générale, il est préférable que le pKa correspondant à la première acidité du solvant soit au moins égal à environ 20 ("environ" soulignant que seul le premier chiffre est significatif), avantageusement au moins égal à environ 25, de préférence compris entre 25 et 35.

Il est préférable que ledit acide ou sel d'acide fluorocarboxylique soit au moins partiellement, de préférence complètement, soluble dans le milieu constituant le réactif.

Les solvants donnant de bons résultats peuvent être notamment des solvants de type amide. Parmi les amides, on comprend aussi les amides à caractère particulier comme les urées tétrasubstituées et les lactames monosubstitués. Les amides sont de préférence substitués (disubstitués pour les amides ordinaires). On peut citer par exemple les dérivés de pyrrolidone, tels que la N-méthylpyrrolidone, ou encore le N,N-diméthylformamide, ou le N,N-diméthylacétamide.

Une autre catégorie particulièrement intéressante de solvants est constituée par les éthers, qu'ils soient symétriques ou non symétriques, qu'ils soient ouverts ou non. Dans la catégorie des éthers doivent être incorporés les différents dérivés des éthers de glycol tels que les différents glymes, le diglyme par exemple.

Il est également apparu que la décomposition des acides fluorocarboxyliques pouvait être obtenue de façon particulièrement efficace lorsque la teneur en hydrogènes labiles du système, ou plus exactement en protons libérables, était inférieure à la teneur en groupes fluorés libérés par la décomposition des sels d'acides fluorocarboxyliques. Par hydrogène labile et proton libérable, on entend un atome d'hydrogène susceptible d'être arraché par une base forte sous forme de proton. En pratique, il s'agit des protons des fonctions acides qui présentent un pKa inférieur à environ 20 (par "environ", on souligne que le nombre 20 ne présente qu'un chiffre significatif).

De préférence, la teneur en protons libérables portés par les divers composants du réactif, y compris leurs impuretés, est au plus égale à la moitié de la concentration molaire initiale en ledit acide fluorocarboxylique.

Plus la teneur en protons libérables dans le réactif sera faible, moins il y aura de risque de réaction parasite et meilleur sera le rendement.

Ainsi, il est préférable que, dans le réactif, la teneur en atomes d'hydrogène labiles soit au plus égale à 10%, de préférence à 1% (en moles), par rapport à la teneur initiale en ledit acide fluorocarboxylique.

La principale impureté, porteuse d'atomes d'hydrogène labiles, est en général l'eau qui est susceptible de libérer jusqu'à deux atomes d'hydrogène par molécule.

D'une manière générale il est préférable d'utiliser des réactifs et des solvants soigneusement déshydratés, de manière que la teneur pondérale en eau du milieu réactionnel soit au plus égale à 1 pour 1000, avantageusement à 5 pour 10000, de préférence à 1 pour 10000. Toutefois, au cours de l'étude qui a mené à la présente invention, il a été montré que, pour maximiser les rendements, il était judicieux de faire en sorte que la teneur en eau ne tombât à zéro. En particulier, on préfère que la teneur en eau soit au moins égale à environ 10 ppm (en masse), avantageusement à environ 50 ppm, de préférence à environ 100 ppm. Ramené à la concentration en acide fluorocarboxylique, il est préférable que la teneur molaire en eau soit au plus égale à environ 5000 ppm, avantageusement à environ 1 pour 1000, de préférence à environ 5 pour 10000. Il est en outre judicieux de faire en sorte que la teneur en eau ne tombe à zéro ; en particulier, on préfère que la teneur en eau (en mole) soit au moins égale à environ 50 ppm, avantageusement à environ 200 ppm par rapport à la concentration en acide fluorocarboxylique.

Par ailleurs, il a pu être montré que d'autres éléments, à savoir les éléments de transition, surtout ceux ayant deux états de valence stables, tels le cuivre ou l'europium, pouvaient n'être pas fastes, voire pouvaient être néfastes (cas notamment du cuivre et des lanthanides à des valences stables) pour l'invention.

Bien que ce réactif selon l'invention ne nécessite pas de catalyseur, de tels éléments métalliques peuvent être présents en tant qu'impuretés apportées notamment par le solvant.

Ainsi, il est préférable que la teneur molaire en ces éléments soit inférieure à 1000, avantageusement à 100, de préférence à 10 ppm par rapport à la teneur initiale en ledit acide fluorocarboxylique.

Egalement, bien qu'il ait été de nombreuses fois préconisé d'utiliser avec l'acide perfluoroacétique des éléments de la colonne VIII de la classification périodique des éléments, pour favoriser certains substrats et favoriser certains types de réaction, cela s'est révélé particulièrement néfaste pour la réaction visée ci-dessus. C'est pourquoi il est préférable d'utiliser des réactifs ne contenant pas de métaux de la colonne VIII, notamment des métaux de la mine du platine qui est le groupe constitué du platine, de l'osmium, de l'iridium, du palladium, du rhodium et du ruthénium.

Dans la présente description, il est fait référence au supplément au bulletin de la Société Chimique de France numéro 1, janvier 1966, où une classification périodique des éléments a été publiée.

Ainsi il est préférable que la teneur en métaux de la mine du platine, voire en métaux de la colonne VIII, soit inférieure à 100 ppm, avantageusement à 10 ppm, de préférence à 1 ppm. Ces valeurs s'entendent par rapport à l'acide fluorocarboxylique de départ et sont exprimées en moles.

D'une manière plus générale et plus empirique, on peut indiquer que ces deux catégories de métaux, à savoir les éléments de transition surtout ceux présentant deux états de valence stables, et les éléments de la colonne VIII, doivent être présents dans le réactif à un niveau de concentration globale au plus égal à 1000 ppm molaires, de préférence à 10 ppm molaires.

On notera que les différents métaux présents à un tel niveau de concentration globale sont en quantité extrêmement faible et, à cet égard, ils ne jouent aucune rôle catalytique. Leur présence n'améliore pas la cinétique de la réaction, voire lui est néfaste lorsqu'ils sont présents en trop grande quantité.

L'utilisation, en plus des composants de réactifs précités, de fluorure de métal alcalin ou de fluorure d'ammonium quaternaire, habituellement présents dans les systèmes réactifs utilisant des carboxylates fluorés, ne s'est pas révélée néfaste, mais elle s'est révélée de peu d'intérêt, notamment en raison du fait qu'elle produit des effluents salins difficiles à traiter. C'est la raison pour laquelle, il est préférable de limiter leur teneur, en particulier leur teneur initiale. Ainsi il est préférable que la teneur en fluorure, que l'on qualifie de ionique, c'est-à-dire susceptible d'être ionisé dans le milieu polarisant du réactif, soit au plus égale à la concentration molaire initiale en ledit sel d'acide fluorocarboxylique, avantageusement à la moitié, de préférence au quart.

Dans l'acide fluorocarboxylique du constituant a) du réactif de l'invention, l'entité Ea qui exerce un effet électroattracteur sur l'atome de carbone difluoré est de préférence choisie parmi les groupes fonctionnels dont la constante de Hammett sₚ est au moins égale à 0,1. Il est en outre préférable que la composante inductive de sₚ, sᵢ, soit au moins égale à 0,2, avantageusement à 0,3. A cet égard, on se référera à l'ouvrage de March, "Advanced Organic Chemistry", troisième édition, John Wiley and Son, pages 242 à 250, et notamment au tableau 4 de cette section.

Plus particulièrement, l'entité électroattractrice peut être choisie parmi les atomes d'halogène, de préférence légers, notamment de chlore et de fluor. L'acide fluorocarboxylique correspondant est un acide halogénofluoroacétique de formule (1)
X - CF₂ - COOH où X est un atome d'halogène, avantageusement léger (chlore ou fluor).

Ea peut également être avantageusement choisie parmi les groupements nitriles, carbonylés, sulfonés et perfluoroalcoylés. Des acides fluorocarboxyliques de ce type qui peuvent être utilisés répondent à la formule (2) R - G - CF₂ - COOH où R - G représente un groupe nitrile ou G représente 〉C = O, 〉S = O, ou
-(CF₂)ₙ- où n est supérieur ou égal à 1, et R représente un résidu organique ou minéral indifférent, de préférence un radical organique tel que aryle, alcoyle, ou aralcoyle, éventuellement substitué. R peut également représenter un support solide minéral ou organique, tel qu'une résine.

Dans le cas où G représente un groupe perfluoroalkylène - (CF₂)ₙ -, n est avantageusement compris entre 1 et 10, de préférence entre 1 et 5. Toujours dans ce cas, R peut également représenter un atome d'halogène, notamment le fluor.

De manière générale, sauf dans le cas où l'acide fluorocarboxylique est un polymère, le nombre total d'atomes de carbone de l'acide fluorocarboxylique, n'excède avantageusement pas 50.

Les contre cations susceptibles de former un sel avec ledit acide fluorocarboxylique sont avantageusement volumineux. Ainsi, on préfère des sels alcalins, avantageusement ceux où le métal alcalin est choisi parmi le sodium, le potassium, le rubidium, le césium et le francium. De préférence, ledit métal est d'une période dont le rang est au moins égal à celle du sodium, avantageusement à celle du potassium. On préfère également des sels d'ammonium quaternaire. A période identique, les alcalinoterreux donnent des résultats similaires à ceux des alcalins.

Il est également possible d'améliorer la réaction en utilisant des cations qui sont, soit naturellement volumineux comme les cations ammonium quaternaire ou phosphonium quaternaire, ou rendus volumineux par l'addition d'agents chélatants ou de préférence cryptands, tels que par exemple les éthers couronne ou les dérivés qui sont à la fois aminés et oxygénés.

Des sels d'acides perfluorocarboxyliques peuvent être avantageusement utilisés, tels que les trifluoroacétate, perfluoropropionate et perfluorobutyrate de métal alcalin, notamment de potassium.

Le réactif réagit selon l'invention avec un composé électrophile, comportant un atome électrophile, cet atome pouvant être un atome de carbone ou un hétéroatome, par exemple le soufre, le sélénium ou le tellure. Il réagit avantageusement avec des composés hydrocarbonés sur un atome de carbone électrophile n'appartenant pas à un système aromatique.

Selon un premier aspect de l'invention, le réactif réagit de préférence avec des composés comportant un atome électrophile, avantageusement un hétéroatome électrophile, lié à un atome notamment d'halogène ou à un groupement pseudohalogène susceptible d'être substitué en une étape.

La réaction marche d'autant mieux que, par opposition avec une SN2, l'on passe par un intermédiaire réactionnel provenant d'une addition sur une liaison multiple ou sur un doublet.

Lorsque l'atome électrophile est un atome de soufre, on peut citer la réaction avec :
- les dérivés halogénés ou pseudohalogénés de composés organiques du soufre, notamment les halogénures de sulfényle, de sulfinyle ou de sulfonyle, où l'atome d'halogène ou le groupement pseudohalogène est substitué au cours de la réaction par un groupement difluorométhyle substitué ;
- des disulfures, par exemple des aryldisulfures éventuellement substitués, où la liaison S-S est rompue et remplacée par un groupement difluorométhyle substitué ; des disulfures appropriés peuvent être notamment des aryldisulfures en C₅-C₁₀, éventuellement substitués par un groupe alkyle en C₁-C₁₀, alcoxy en C₁-C₁₀, nitro ou par un ou plusieurs (£ 3) atomes d'halogène ;
- les oxydes de soufre, comme le bioxyde de soufre ;
- les composés de type thiocyanate où le groupe cyano est substitué au cours de la réaction par un groupement difluorométhyle substitué ; des thiocyanates préférés sont les thiocyanates d'aryle en C₅-C₁₀, y compris d'alcoylaryle, et thiocyanates d'alcoyle en C₁-C₁₀, y compris d'aralcoyle.

Dans les composés ci-dessus, l'atome d'halogène peut être choisi parmi les atomes d'iode, de brome, de chlore et de fluor. Un groupement "pseudohalogène" est un groupe qui, partant, sous forme anionique, présente un acide associé dont le pKa est inférieur à 4, de préférence à 3, en particulier à 0.

On préfère les groupes dont l'acide associé présente une acidité (mesurée par la constante de Hammett) au moins égale à celle de l'acide acétique, avantageusement à celle des acides sulfoniques, ou des acides trihalogénés. Un des pseudohalogènes typiques est un groupe perfluoroalcanesulfonyloxyle qui libère un perfluoroalcanesulfonate. Des groupes pseudohalogènes préférés peuvent être choisis parmi les groupe tosylate (p-toluènesulfonyloxyle), mésylate (méthylsulfonyloxyle), trifluorométhylsulfonyloxyle ou trifluoroacétoxyle. On peut également considérer le groupe acétate comme un tel groupe partant.

Parmi ces groupes, les groupes perfluoro-alcanoyloxyles tels que trifluoroacétyloxyle sont très avantageux dans le cas où l'on souhaite greffer un groupe perfluoroalcoyle (trifluorométhyle dans le cas où le groupe initial est le groupe trifluroacétyloxyle) . Par exemple, la substitution du groupe trifluoroacetyloxyle régénère in situ le réactif en libérant dans le milieu de l'acide trifluoroacétique ou un de ses sels, qui peut à son tour réagir comme source de groupements trifluorométhyle.

Selon un autre aspect, le réactif réagit également avantageusement sur un composé choisi parmi les composés carbonylés de type cétone, aldéhyde, halogénure d'acide ou ester activé, en réalisant une addition sur la fonction carbonyle.

On pourra citer à titre d'exemples préférentiels et non limitatifs des aldéhydes aromatiques, de préférence en C₅-C₁₀, où le noyau aromatique peut éventuellement être substitué par un groupe alkyle en C₁-C₁₀, alcoxy en C₁-C₁₀, nitro ou par un atome d'halogène ; des cétones cycliques comme la cyclohexanone ; des cétones non énolisables activées par un groupe donneur, comme la trifluorométhylacétophénone; des anhydrides aromatiques, comme l'anhydride benzoïque.

Le produit de la réaction est en général dans ce cas un alcool (par exemple sous forme d'alcoolate) dont l'atome de carbone porteur de la fonction hydroxyle est substitué par un groupement difluorométhyle substitué. Ce produit peut éventuellement réagir ensuite avec le réactif ou avec le produit de départ suivant les conditions réactionnelles.

De manière générale, la quantité de réactif engagé dans le procédé de l'invention sera fixée de manière connue en soi selon la fonctionnalité du composé électrophile.

Il est en général préférable que le rapport soit compris entre 1 et 10, avantageusement aux alentours de deux, fonctions électrophiles par molécule d'acide fluorocarboxylique.

Il convient de signaler que le produit issu de la décomposition de l'acide fluorocarboxylique peut réagir sur lui-même s'il comporte une des fonctions susceptibles de réagir.

On pourra noter que des composés à fonction électrophile se présentant sous forme liquide sont susceptibles d'être utilisés en tant que solvant selon la présente invention dans la mesure où ils sont aprotiques. On peut ainsi réaliser avantageusement la réaction de la présente invention en mettant en présence
a) un sel d'acide fluorocarboxylique tel que défini précédemment et
b) un composé comportant au moins une fonction électrophile agissant à la fois comme solvant et comme substrat de la réaction.

Lors de la mise en oeuvre du réactif selon l'invention avec un substrat comportant au moins une fonction électrophile, il est important que ce dernier perturbe le moins possible les conditions décrites précédemment.

Ainsi, il est préférable d'utiliser un substrat suffisamment déshydraté, ou qui ne comporte pas d'hydrogène acide arrachable par des bases fortes ni d'impuretés néfastes c'est-à-dire, d'une manière générale, qui satisfasse les mêmes contraintes que celles exposées pour le réactif.

Un des aspects de la présente invention concerne plus particulièrement l'application du procédé revendiqué à la préparation de dérivés organiques oxysulfurés et fluorés à partir d'un oxyde soufre, notamment du bioxyde de soufre. Il permet notamment de former des acides sulfiniques ou sulfoniques fluorés.

Au cours de l'étude qui a mené à la présente invention, il a ainsi été démontré qu'il était possible de greffer sur un oxyde de soufre des radicaux fluoroalcoyle générés à partir d'un acide fluorocarboxylique, sans catalyseur et sans agent susceptible de stabiliser les divers intermédiaires envisagés obtenus lors de la décomposition des différents acides perfluoro-carboxyliques, avec un rendement et une sélectivité de transformtion satisfaisants et une cinétique remarquablement élevée en travaillant dans un solvant aprotique polaire et sous l'action de micro-ondes.

Dans ce cas particulier, il est préférable que le rapport des quantités relatives dudit acide fluorocarboxylique initial et d'oxyde de soufre de préférence bioxyde, soit compris entre 1 et 10, avantageusement aux alentours de deux atomes de soufre par molécule d'acide fluorocarboxylique.

Lorsque ledit oxyde est du bioxyde de soufre, le mélange résultant de l'étape a) peut comprendre deux phases en équilibre et comporter ainsi une phase liquide, où une partie au moins dudit acide et du bioxyde de soufre sont dissous dans ledit solvant, en équilibre avec une phase gazeuse qui contient du bioxyde de soufre.

Toujours dans le cas du bioxyde de soufre, le produit obtenu par chauffage du réactif est un acide sulfinique ou un sel d'acide sulfinique dont le contre-ion est celui du sel d'acide fluorocarboxylique de départ.

Pour passer de l'acide sulfinique à l'acide sulfonique correspondant, il convient de soumettre le produit réactionnel ou le produit de réaction purifié à une oxydation, en elle-même connue, notamment au moyen d'eau oxygénée ou d'hypochlorite de sodium. Un procédé de purification de trifluorométhylsulfinate de sodium, et d'oxydation en sulfonate, applicable selon l'invention est décrit dans la demande de brevet européen publiée sous le numéro EP-A-0 396 458. Les sels d'acides sulfiniques ou sulfoniques ainsi obtenus peuvent être convertis en les acides libres correspondants en milieu acide. Les produits de réaction, sels ou acides libres, peuvent être isolés aisément et mis en oeuvre dans des étapes ultérieures de synthèse organique. Ainsi, par exemple, on peut préparer à partir d'acides sulfiniques fluorés les chlorures de sulfinyle correspondants. Le second élément essentiel de l'invention consiste à amener le réactif à réagir avec le composé électrophile sous l'action de micro-ondes.

L'exposition du milieu réactionnel à l'action de micro-ondes permet d'activer le milieu, de manière comparable à la température au sein même de ce dernier. Il est apparu que cette activation procure une amélioration remarquable de la cinétique de réaction. Selon la puissance appliquée, le temps de réaction peut varier notamment de 30 secondes à 1 heure, en particulier de 1 à 30 minutes.

Il est difficile de définir une température d'un milieu pendant l'exposition au micro-ondes. On peut toutefois indiquer que la température du milieu avant l'action des micro-ondes est avantageusement comprise entre la température de congélation commençante du milieu et 110°C.

On peut également faire circuler le milieu soumis au micro-ondes dans un dispositif réfrigérant de manière à maintenir la température du milieu réactionnel en sortie de la zone d'exposition aux micro-ondes au plus égale à 150°C, avantageusement à 130°C, de préférence à 120°C.

L'exposition du milieu réactionnel aux micro-ondes est avantageusement telle que le milieu soit soumis au rayonnement avec une énergie au moins égale à 1 Watt par seconde et par kg de masse réactionnelle, de préférence au moins égale à 5 W/s/kg.

Lorsque le produit d'arrivée est fragile, il est préconisé que l'énergie rayonnée soit au plus égale à environ 100 W/s/kg, avantageusement à environ 50 W/s/kg, de préférence à environ 20 W/s/kg.

La fréquence des micro-ondes utilisable est comprise entre environ 100 MHz et environ 10 GHz, avantageusement entre environ 300 MHz et 3 GHz. La longueur d'onde des micro-ondes utilisable est généralement comprise entre 10 cm et 1 m dans l'air.

On appliquera avantageusement les micro-ondes avec une durée et une intensité correspondant à au moins 5 Watts par seconde et par mole d'acide fluorocarboxylique, de préférence au moins environ 20 W/s/mol.

Lorsque le produit d'arrivée est fragile, il est préconisé que l'énergie rayonnée soit au plus égale à 1000 W/s/mole d'acide fluorocarboxylique, avantageusement à environ 500 W/s/mole d'acide fluorocarboxylique, de préférence à environ 200 W/s/mole d'acide fluorocarboxylique.

Dans le cas particulier, où le procédé revendiqué est appliqué à la préparation de dérivés organiques oxysulfurés et fluorés comme décrit précédemment, on appliquera avantageusement les micro-ondes avec une durée et une intensité correspondant à au moins 5 Watts par seconde et par mole d'acide fluorocarboxylique, de préférence au moins environ 20 W/s/mol.

L'application des micro-ondes au milieu réactionnel peut se faire par tout moyen connu en soi.

Avantageusement, l'applicateur de micro-ondes se présente sous la forme d'une cavité (réacteur) renfermant le milieu réactionnel. On peut également disposer à l'intérieur du réacteur des éléments internes fortement dissipatifs sous rayonnement de micro-ondes et transmettant l'énergie au milieu réactionnel.

La géométrie du dispositif sera avantageusement définie en fonction des caractéristiques de dissipation de l'énergie par le milieu réactionnel.

De préférence, on excitera un seul mode d'onde pour maîtriser au mieux la dissipation d'énergie. On préférera alors des réacteurs conçus pour des procédés en continu dont la géométrie simple (le plus souvent cylindrique) est adaptée à la transmission monomode des ondes. Ces réacteurs continus peuvent fonctionner avec un recyclage du milieu réactionnel.

L'applicateur peut notamment se présenter sous la forme d'un réacteur tubulaire disposé parallèlement à un guide d'ondes à fentes rayonnantes.

Néanmoins, des réacteurs de type "batch" pour procédés discontinus peuvent également être utilisés pour exposer le milieu réactionnel aux micro-ondes en monomode. L'homogénéité thermique du milieu réactionnel est avantageusement assurée par la rotation du réacteur et/ou par l'agitation du milieu à contre sens par un agitateur en verre à pales.

L'exposition aux micro-ondes pourra éventuellement être couplée à un mode de chauffage classique.

Les exemples suivants illustrent l'invention.

### Exemple 1 :

### Préparation du phényltrifluorométhylsulfure.

Dans un réacteur en téflon de 30 ml, on charge 2,26 g (14,9 mmol) de trifluoroacétate de potassium anhydre, 1,6 g (7,4 mmol) de phénylsulfure et 17 g de diméthylformamide (DMF) anhydre.

Le rapport molaire du trifluoroacétate de potassium au phényldisulfure est de 2,0.

La teneur en eau du milieu réactionnel est inférieure à 0,002 % en moles par rapport au trifluoroacétate.

Le réacteur est fermé puis placé dans un four à micro-ondes de puissance maximale 300 W avec un système monomode à une fréquence de 2450 MHz, les micro-ondes étant focalisées à la base du réacteur à l'aide d'un guide d'onde.

On procède ensuite à l'émission de micro-ondes pendant 10 minutes à raison de 2,5 W/s/kg de masse réactionnelle (puissance cumulée : environ 30 W), puis pendant 5 minutes à raison de 10 W/s/kg (puissance cumulée dans cette seconde étape : environ 60 W).

Après refroidissement, la masse réactionnelle est analysée par chromatographie en phase gazeuse et RMN du ¹⁹F.

On détermine que le taux de transformation du disulfure (quantité de disulfure disparue/quantité de disulfure initiale) vaut 69 %.

Le rendement réel en phényltrifluorométhylsulfure (quantité de phényltrifluorométhylsulfure formée par rapport à la quantité de phényldisulfure initiale) est de 55,4 %, ce qui correspond à une sélectivité exprimée par le rendement de transformation du disulfure (quantité de phényltrifluorométhylsulfure formée par rapport à la quantité de phényldisulfure transformée), de 80,4 %.

On détermine de la même façon que le taux de transformation du trifluoroacétate de potassium est de 62 % et que la sélectivité de transformation en phényltrifluorométhylsulfure est de 87,5 %.

### Exemple comparatif 1 :

La même réaction est conduite sans recours aux micro-ondes.

Dans un tube de verre de 30 ml avec agitation magnétique, les réactifs sont chargés comme dans l'exemple 1, puis chauffés pendant 28 heures à 140°C.

Après refroidissement, le mélange est dosé par chromatographie gazeuse pour déterminer les résultats suivants :
- taux de transformation du phényldisulfure: 67 %
- rendement de transformation de phényldisulfure en phényltrifluorométhylsulfure (sélectivité) : 76%.

On constate que l'on atteint en quelques minutes sous micro-ondes un taux de transformation analogue à celui obtenu en plus d'une journée avec chauffage traditionnel, avec un gain de sélectivité de près de 5 %. Le procédé de l'invention est donc bien plus performant qu'un procédé à chauffage traditionnel.

### EXEMPLE 2 :

### Préparation d'acide trifluorométhylsulfinique.

Dans un réacteur tubulaire en téflon de 30 ml, surmonté d'un manomètre allant de 0 à 20 bars, et d'une vanne de dégazage, on introduit 13 g de N-méthylpyrrolidone (NMP ; teneur en eau inférieure à 10 ppm en poids), 1,6 g de CF₃CO₂K (teneur en eau inférieure à 100 ppm en poids) et environ 2 g de bioxyde de soufre gazeux (teneur en eau inférieure à 0,01 % molaire) par barbotage dans le liquide.

Le rapport molaire CF₃CO₂K/SO₂ est d'environ 2. Le rapport massique CF₃CO₂K/SO₂ est d'environ 0,13.

Ce réacteur fermé est introduit dans un four à micro-ondes de puissance maximale 300 W avec un système monomode à une fréquence de 2 450 MHz, les micro-ondes étant focalisées à la base du réacteur à l'aide d'un guide d'onde.

On procède ensuite à l'émission de micro-ondes pendant 10 minutes à raison de 10 W/s/kg de masse réactionnelle, soit une puissance totale de 30 W environ. La pression passe de 0 à 2 bars (2.10⁵ Pa) au cours de la réaction pour retomber à 0 après arrêt des micro-ondes et refroidissement du milieu réactionnel.

Le mélange réactionnel est ensuite repris à l'eau et analysé par RMN du ¹⁹F et chromatographie ionique HPIC (high performance ionic chromatography) en mode de séparation.

On détermine le taux de transformation, le rendement réel en CF₃SO₂K et le rendement de transformation qui sont indiqués dans le tableau 1 ci-après pour les exemples 1 à 6.

### EXEMPLE 3 :

On reproduit l'exemple 2 en respectant un rapport CF₃CO₂K/SO₂ entre 1,9 et 2,1 et en appliquant les micro-ondes pendant 7 minutes à raison de 10 W/s/kg pour une puissance totale de 45 W. La pression dans le réacteur passe par un maximum de 3,8.10⁵ Pa au cours de la réaction.

### EXEMPLE 4 :

On reproduit l'exemple 2 en respectant un rapport CF₃SO₂K/SO₂ entre 1,9 et 2,1 en imposant les micro-ondes pendant 4 minutes à raison de 10 W/s/kg pour une puissance totale de 60 W. La pression dans le réacteur passe par un maximum de 3.10⁵ Pa au cours de la réaction.

### Exemple comparatif 2 :

### Préparation d'acide trifluorométhylsulfinique sans recours aux micro-ondes.

Dans un réacteur en Hastalloy de 100 ml, agité par une turbine, on introduit 42 g de N-méthylpyrrolidone (NMP), puis 5,32 g (35 mmol) de trifluoroacétate de potassium et enfin 4,9 g (76 mmol) de bioxyde de soufre gazeux par barbotage dans le liquide. Le bioxyde de soufre est totalement solubilisé par la NMP.

Le rapport molaire du bioxyde de soufre au trifluoroacétate de potassium est de 1,5.

La teneur en eau du mélange réactif est de 0,1 % en poids par rapport au poids du mélange, soit un rapport molaire de l'eau au trifluoroacétate de 0,07.

Le mélange est chauffé dans le réacteur fermé à une température de 140°C pendant 6 heures sous agitation.

Au cours de la réaction, la pression à l'intérieur du réacteur ramené à une température ambiante s'élève de 3,5.10⁵ Pa par rapport à la pression initiale.

Le milieu réactionnel est ensuite repris à l'eau et analysé par RMN du ¹⁹ F pour doser la transformation du trifluoroacétate de potassium.

Le taux de transformation (TT) du trifluoroacétate de potassium de départ, exprimé par le rapport molaire de la quantité de trifluoroacétate consommée (transformée) à la quantité initiale, est de 61,7 %.

Le rendement réel (RR), exprimé par le rapport molaire de la quantité de trifluorométhylsulfinate formée, sous forme libre ou salifiée, à la quantité de trifluoroacétate initiale, est de 29,7 %.

Le rendement par rapport au produit transformé (RT), exprimé par le rapport molaire de la quantité de trifluorométhylsulfinate formée, sous forme libre ou salifiée, à la quantité de trifluoroacétate transformée, est de 48,1 %. On isole le produit sous forme de sel de potassium.

### EXEMPLES 5-7 :

Ces exemples illustrent l'effet des micro-ondes lorsque la réaction de l'exemple 2 est effectuée dans le diméthylformamide (DMF).

Les conditions opératoires générales sont identiques hormis que la NMP est remplacée par du DMF.

Trois conditions différentes sont appliquées pour les micro-ondes :
- exemple 5 : 7 minutes pour une puissance totale de 45 W ;
- exemple 6: 7 minutes pour une puissance totale de 45 W avec ajout d'un équivalent molaire de KF par rapport à SO₂;
- exemple 7 : 5 minutes pour une puissance totale de 60 W.

Les résultats sont récapitulés dans le tableau 1 ci-après.

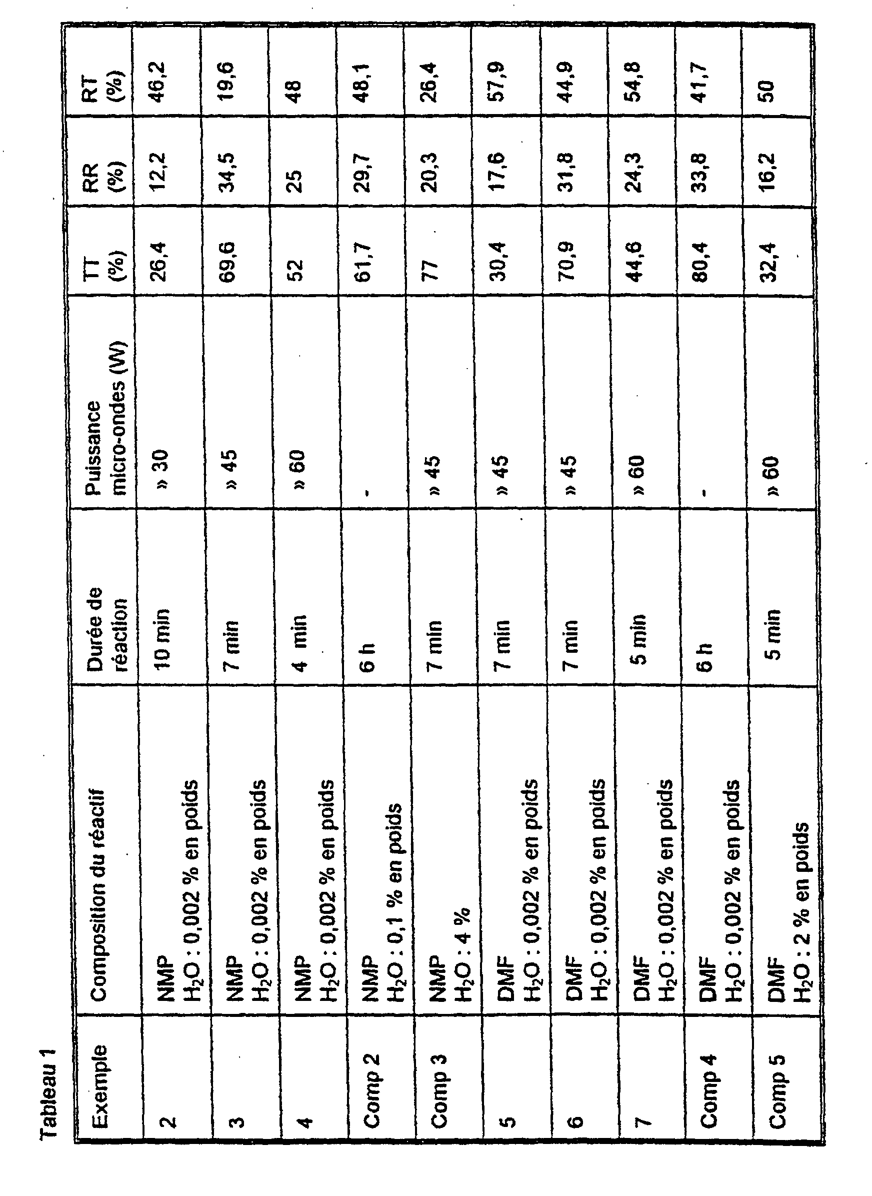

Dans tous les exemples selon l'invention, on constate que l'on atteint en quelques minutes sous micro-ondes des résultats analogues à ceux obtenus en 6 heures par simple chauffage (exemple comparatif 4).

Les exemples comparatifs 3 et 5 montrent que la présence d'eau en quantité importante dans le milieu réactionnel nuit à l'obtention de trifluorométhylsulfinate de potassium en quantité satisfaisante.

Les conditions décrites dans les exemples 2 à 7 sont adaptées à la préparation d'autres composés oxysulfurés tels que notamment l'acide pentafluoroéthylsulfinique (à partir de C₂F₅COOK) ou l'acide heptafluoropropylsulfinique (à partir de C₃F₇COOK) sous forme de sulfinate de potassium.

### Exemple 8 :

### Préparation de chlorure de trifluorométhylsulfinyle

On prépare du trifluoroacétate de potassium dans les conditions de l'exemple 7.

Le DMF est éliminé du mélange réactionnel par distillation sous vide à une température ne dépassant pas 55-60°C.

Le résidu de distillation est repris à l'acétonitrile puis filtré. Le filtrat est distillé pour éliminer le solvant et on isole le trifluoroacétate de potassium avec un rendement de purification de 96 % par rapport au mélange réactionnel brut, dosé par chromatographie ionique.

Le produit résultant de cette opération est repris au toluène et additionné de chlorure de thionyle SO₂Cl₂ en quantité stoechiométrique par rapport au trifluorométhylsulfinate. Le chlorure de trifluorométhylsulfinyle est obtenu avec un rendement de 65 %.

## Revendications

1. Procédé pour greffer un groupement difluorométhyle substitué sur un composé comportant au moins une fonction électrophile, comprenant les étapes consistant à:
i) mettre ledit composé comportant au moins une fonction électrophile en présence d'un réactif nucléophile comprenant:
a) un acide fluorocarboxylique de formule Ea - CF₂ - COOH où Ea représente un atome ou un groupe électroattracteur, au moins partiellement salifié par un cation organique ou minéral, et
b) un solvant aprotique polaire ; et
ii) exposer le milieu réactionnel à l'action de micro-ondes.

2. Procédé selon l'une quelconque des revendications précédentes, dans lequel la teneur en protons libérables portés par les divers composants du réactif, y compris leurs impuretés, est au plus égale à la moitié de la concentration molaire initiale dudit acide fluorocarboxylique.

3. Procédé selon la revendication 2, dans lequel ladite teneur en protons est au plus égale à 10 % de la concentration molaire initiale en ledit sel d'acide fluorocarboxylique.

4. Procédé selon l'une quelconque des revendications précédentes, dans lequel la teneur pondérale en eau du milieu réactionnel est au plus égale à 1 pour 1000.

5. Procédé selon l'une quelconque des revendications précédentes, dans lequel la teneur en eau du milieu réactionnel est au moins égale à 10 ppm en poids.

6. Procédé selon l'une quelconque des revendications précédentes, dans lequel le rapport molaire de la teneur en eau à la concentration en acide fluorocarboxylique est au plus égal à 5000 ppm.

7. Procédé selon l'une quelconque des revendications précédentes, dans lequel le rapport molaire de la teneur en eau à la concentration en acide fluorocarboxylique est au moins égal à 50 ppm.

8. Procédé selon l'une quelconque des revendications précédentes, dans lequel la teneur en éléments de transition dudit réactif est inférieure à 1000 ppm molaires, par rapport audit sel d'acide fluorocarboxylique.

9. Procédé selon l'une quelconque des revendications précédentes, dans lequel la teneur en éléments de la colonne VIII de la classification périodique des éléments dans ledit réactif est inférieure à 100 ppm molaires, par rapport audit acide fluorocarboxylique.

10. Procédé selon l'une quelconque des revendications précédentes, dans lequel la teneur, exprimée en équivalent, en fluorure ionique dans ledit réactif est au plus égale à la concentration molaire initiale dudit acide fluorocarboxylique.

11. Procédé selon l'une quelconque des revendications précédentes, dans lequel l'indice donneur dudit solvant aprotique polaire est compris entre 10 et 30.

12. Procédé selon l'une quelconque des revendications précédentes, dans lequel le pKa correspondant à la première acidité dudit solvant est au moins égal à 20.

13. Procédé selon l'une quelconque des revendications précédentes, dans lequel ledit atome ou groupement électroattracteur est choisi parmi les groupes électroattracteurs dont la constante de Hammet sₚ est au moins égale à 1.

14. Procédé selon l'une quelconque des revendications précédentes, dans lequel ledit acide est choisi parmi les composés de formule (1) X - CF₂ - COOH, où X représente un atome d'halogène, et les composés de formule (2) R - G - CF₂ - COOH, où R - G représente un groupe nitrile ou G représente 〉C = 0, 〉S = 0 ou -(CF₂)ₙ- avec n supérieur ou égal à 1 et R représente un résidu organique ou minéral indifférent.

15. Procédé selon l'une quelconque des revendications précédentes, dans lequel ledit acide ou sel d'acide fluorocarboxylique est complètement soluble dans le milieu réactif.

16. Procédé selon l'une quelconque des revendications précédentes, dans lequel ledit sel d'acide est un sel de métal alcalin choisi parmi le sodium, le potassium, le rubidium, le césium et le francium, ou un sel d'ammonium quaternaire.

17. Procédé selon l'une quelconque des revendications précédentes, dans lequel le solvant est choisi parmi les amides N-disubstitués, y compris les urées tétrasubstituées et les lactames monosubstitués, et les éthers cycliques ou non.

18. Procédé selon l'une quelconque des revendications précédentes, dans lequel ledit composé à fonction électrophile est choisi parmi les dérivés halogénés ou pseuhalogénés de composés organiques du soufre, tels que les halogénures de sulfényle, de sulfinyle ou de sulfonyle ; les oxydes de soufre notamment des bioxyde de soufre ; les thiocyanates ; les disulfures ; et les composés carbonylés de type cétone, aldéhyde, halogénure d'acide ou ester activé.

19. Procédé selon l'une quelconque des revendications précédentes, dans lequel ledit composé comportant au moins une fonction électrophile ne comporte pas d'hydrogène arrachable par une base forte.

20. Procédé selon l'une quelconque des revendications précédentes, dans lequel la puissance de micro-ondes appliquée est d'au moins 1 Watt par seconde et par kg de masse réactionnelle.

21. Procédé selon l'une quelconque des revendications précédentes, dans lequel la puissance de micro-ondes appliquée est d'au moins 5 Watt par seconde et par mole d'acide fluorocarboxylique.

22. Procédé selon l'une quelconque des revendications précédentes, dans lequel la puissance de micro-ondes appliquée est d'au. plus 100 Watt par seconde et par kg de masse réactionnelle.

23. Procédé selon l'une quelconque des revendications précédentes, dans lequel la température du milieu réactionnel en sortie de la zone d'exposition aux micro-ondes est maintenue au plus égale à 150^{m}C.

24. Procédé selon l'une quelconque des revendications précédentes, dans lequel ledit composé à fonction électrophile est du bioxyde de soufre.

25. Procédé selon la revendication 24, dans lequel le milieu réactionnel de l'étape a) est un liquide en équilibre avec une phase gazeuse contenant du bioxyde de soufre.

26. Procédé selon la revendication 24 ou 25, qui comporte en outre une étape c) d'oxydation du sel d'acide sulfinique obtenu à l'étape b) par mise en présence du produit de l'étape b) avec un réactif d'oxydation.

27. Procédé pour greffer un groupement difluorométhyle substitué sur un composé comportant au moins une fonction électrophile comprenant les étapes consistant à :
i) mettre ledit composé comportant au moins une fonction électrophile en présence d'un réactif nucléophile comprenant un acide fluorocarboxylique de formule Ea-CF₂-COOH où Ea représente un atome ou un groupe électroattracteur, au moins partiellement salifié par un cation organique ou minéral, ledit composé comportant au moins une fonction électrophile agissant à la fois comme solvant et comme substrat de réaction ; et
ii) exposer le milieu réactionnel à l'action de micro-ondes.

28. Application d'un procédé selon l'une quelconque des revendications précédentes à la préparation de dérivés organiques oxysulfurés et fluorés.

## Patentansprüche

1. Verfahren zum Anbringen einer substituierten Difluormethylgruppe an einer Verbindung, die mindestens eine elektrophile funktionelle Gruppe enthält, das die Stufen umfasst, die darin bestehen:
I) die Verbindung, die mindestens eine elektrophile funktionelle Gruppe enthält, mit einem nukleophilen Reagenz, das
a) eine Fluorcarbonsäure mit der Formel Ea-CF₂-COOH, worin Ea ein Elektronen anziehendes Atom oder eine Elektronen anziehende Gruppe bedeutet und welche wenigstens teilweise mit einem organischen oder anorganischen Kation ein Salz bildet, und
b) ein polares aprotisches Lösungsmittel umfasst, in Berührung zu bringen und
II) das Reaktionsmedium der Einwirkung von Mikrowellen auszusetzen.

2. Verfahren nach Anspruch 1, in welchem der Gehalt an freisetzbaren Protonen, die von den verschiedenen Bestandteilen des Reagenz getragen werden, wobei deren Verunreinigungen eingeschlossen sind, höchstens gleich der halben molaren Anfangskonzentration der Fluorcarbonsäure ist.

3. Verfahren nach Anspruch 2, in welchem der Protonengehalt höchstens 10 % der molaren Anfangskonzentration des Fluorcarbonsäuresalzes beträgt.

4. Verfahren nach einem der vorhergehenden Ansprüche, in welchem der Gewichtsanteil des Wassers am Reaktionsmedium höchstens 1 auf 1 000 beträgt.

5. Verfahren nach einem der vorhergehenden Ansprüche, in welchem der Gewichtsanteil des Wassers am Reaktionsmedium mindestens 10 ppm beträgt.

6. Verfahren nach einem der vorhergehenden Ansprüche, in welchem das Molverhältnis von Wassergehalt zur Fluorcarbonsäure-Konzentration höchstens 5 000 ppm beträgt.

7. Verfahren nach einem der vorhergehenden Ansprüche, in welchem das Molverhältnis von Wassergehalt zur Fluorcarbonsäure-Konzentration mindestens 50 ppm beträgt.

8. Verfahren nach einem der vorhergehenden Ansprüche, in welchem der Gehalt an Übergangselementen des Reagenz weniger als 1 000 Mol-ppm, bezogen auf das Fluorcarbonsäuresalz, beträgt.

9. Verfahren nach einem der vorhergehenden Ansprüche, in welchem der Gehalt an Elementen der Gruppe VIII des Periodensystems der Elemente im Reagenz weniger als 100 Mol-ppm, bezogen auf die Fluorcarbonsäure, beträgt.

10. Verfahren nach einem der vorhergehenden Ansprüche, in welchem der als Äquivalent ausgedrückte Gehalt an Fluorid-Ion im Reagenz höchstens gleich der molaren Anfangskonzentration der Fluorcarbonsäure ist.

11. Verfahren nach einem der vorhergehenden Ansprüche, in welchem die Donorzahl des polaren aprotischen Lösungsmittels 10 bis 30 beträgt.

12. Verfahren nach einem der vorhergehenden Ansprüche, in welchem der der ersten Acidität des Lösungsmittels entsprechende pKₐ mindestens 20 beträgt.

13. verfahren nach einem der vorhergehenden Ansprüche, in welchem das Elektronen anziehende Atom oder die Elektronen anziehende Gruppe aus den Elektronen anziehenden Gruppen ausgewählt ist, deren Hammett-Konstante σᵢ mindestens 1 beträgt.

14. Verfahren nach einem der vorhergehenden Ansprüche, in welchem die Säure aus den Verbindungen mit der Formel (1) X-CF₂-COOH, worin X ein Halogenatom bedeutet, und den Verbindungen mit der Formel (2) R-G-CF₂-COOH, worin R-G eine Nitrilgruppe oder G C=O, S=O oder -(CF₂)ₙ-, worin n größer oder gleich 1 ist, und R einen beliebigen organischen bzw. anorganischen Rest bedeutet, ausgewählt ist.

15. Verfahren nach einem der vorhergehenden Ansprüche, in welchem die Fluorcarbonsäure oder das Fluorcarbonsäuresalz vollständig im Reaktionsmedium löslich ist.

16. Verfahren nach einem der vorhergehenden Ansprüche, in welchem das Säuresalz das Salz eines Alkalimetalls, das aus Natrium, Kalium, Rubidium, Cäsium und Franzium ausgewählt ist, oder ein quaternäres Ammoniumsalz ist.

17. Verfahren nach einem der vorhergehenden Ansprüche, in welchem das Lösungsmittel aus disubstituierten N-Amiden, worin tetrasubstituierter Harnstoff und monosubstituierte Lactame eingeschlossen sind, und cyclischen oder anderen Ethern ausgewählt ist.

18. Verfahren nach einem der vorhergehenden Ansprüche, in welchem die Verbindung mit elektrophiler funktioneller Gruppe aus halogenierten oder pseudohalogenierten Derivaten organischer Schwefelverbindungen wie Sulfenyl-, Sulfinyl- oder Sulfonylhalogeniden, Schwefeloxiden, insbesondere Schwefeldioxid, Thiocyanaten und Disulfiden und Carbonylverbindungen vom Typ Keton, Aldehyd, Säurehalogenid oder aktivierter Ester ausgewählt ist.

19. Verfahren nach einem der vorhergehenden Ansprüche, in welchem die Verbindung, die mindestens eine elektrophile funktionelle Gruppe umfasst, keinen Wasserstoff enthält, den eine starke Base abspalten kann.

20. Verfahren nach einem der vorhergehenden Ansprüche, in welchem die eingebrachte Mikrowellenleistung mindestens 1 Watt pro Sekunde und kg Reaktionsmasse beträgt.

21. Verfahren nach einem der vorhergehenden Ansprüche, in welchem die eingebrachte Mikrowellenleistung mindestens 5 Watt pro Sekunde und Mol Fluorcarbonsäure beträgt.

22. Verfahren nach einem der vorhergehenden Ansprüche, in welchem die eingebrachte Mikrowellenleistung höchstens 100 Watt pro Sekunde und kg Reaktionsmasse beträgt.

23. Verfahren nach einem der vorhergehenden Ansprüche, in welchem die Temperatur des Reaktionsmediums nach der Zone der Einstrahlung der Mikrowellen auf höchstens 150 °C gehalten wird.

24. Verfahren nach einem der vorhergehenden Ansprüche, in welchem die Verbindung mit elektrophiler funktioneller Gruppe Schwefeldioxid ist.

25. Verfahren nach Anspruch 24, in welchem das Reaktionsmedium der Stufe a) eine Flüssigkeit im Gleichgewicht mit einer Schwefeldioxid-haltigen Gasphase ist.

26. Verfahren nach Anspruch 24 oder 25, das außerdem eine Stufe c) der Oxidation des in Stufe b) erhaltenen Sulfinsäuresalzes durch In-Berührung-Bringen des Produkts von Stufe b) mit einem Oxidationsmittel umfasst.

27. Verfahren zum Anbringen einer substituierten Difluormethylgruppe an einer Verbindung, die mindestens eine elektrophile funktionelle Gruppe enthält, das die Stufen umfasst, die darin bestehen:
I) die Verbindung, die mindestens eine elektrophile funktionelle Gruppe enthält, mit einem nukleophilen Reagenz in Berührung zu bringen, das eine Fluorcarbonsäure mit der Formel Ea-CF₂-COOH umfasst, worin Ea ein Elektronen anziehendes Atom oder eine Elektronen anziehende Gruppe bedeutet und welche wenigstens teilweise mit einem organischen oder anorganischen Kation ein Salz bildet, wobei diese Verbindung, die mindestens eine elektrophile funktionelle Gruppe enthält, gleichzeitig als Lösungsmittel und Reaktionssubstrat agiert, und
II) das Reaktionsmedium der Einwirkung von Mikrowellen auszusetzen.

28. Anwendung eines Verfahrens nach einem der vorhergehenden Ansprüche für die Herstellung oxysulfurierter und fluorierter organischer Verbindungen.

## Claims

1. Process for grafting a substituted difluoromethyl group onto a compound having at least one electrophilic function, comprising the steps of:
i) bringing said compound having at least one electrophilic function into contact with a nucleophilic reagent comprising:
a) a fluorocarboxylic acid of formula Ea-CF₂-COOH where Ea denotes an electron-attracting atom or group, at least partially salified by an organic or inorganic cation, and
b) a polar aprotic solvent; and
ii) exposing the reaction mixture to the action of microwaves.

2. Process according to any of the preceding claims, wherein the content of releasable protons carried by the various components of the reagent, including their impurities, is equal to not more than half the initial molar concentration of said fluorocarboxylic acid.

3. Process according to claim 2, wherein said proton content is at most equal to 10% of the initial molar concentration of said fluorocarboxylic acid salt.

4. Process according to any one of the preceding claims, wherein the water content, by weight, of the reaction mixture is equal to not more than 1 per 1000.

5. Process according to any one of the preceding claims, wherein the water content of the reaction mixture is equal to at least 10 ppm by weight.

6. Process according to any one of the preceding claims, wherein the molar ratio of the water content to the concentration of fluorocarboxylic acid is equal to

7. Process according to any one of the preceding claims, wherein the molar ratio of the water content to the concentration of fluorocarboxylic acid is equal to at least 50 ppm.

8. Process according to any one of the preceding claims, wherein the content of transition elements in said reagent is less than 1000 molar ppm, in relation to said fluorocarboxylic acid salt.

9. Process according to any one of the preceding claims, wherein the content of elements from column VIII of the Periodic Table of Elements in said reagent is less than 100 molar ppm, in relation to said fluorocarboxylic acid salt.

10. Process according to any one of the preceding claims, wherein the content, expressed as equivalents, of ionic fluoride in said reagent is at most equal to the initial molar concentration of said fluorocarboxylic acid salt.

11. Process according to any one of the preceding claims, wherein the donor index of said polar aprotic solvent is between 10 and 30.

12. Process according to any one of the preceding claims, wherein the pKa corresponding to the first acidity of said solvent is equal to at least 20.

13. Process according to any one of the preceding claims, wherein said electron-attracting atom or group is selected from among the electron-attracting groups having a Hammett constant σₚ of at least 0.1.

14. Process according to any one of the preceding claims, wherein said acid is selected from among the compounds of formula (1) X - CF₂ - COOH, where x denotes a halogen atom, and the compounds of formula (2) R - G - CF₂ - COOH, where R - G denotes a nitrile group or G denotes C=0, S=0 or -(CF₂)ₙ- where n is greater than or equal to 1 and R denotes an inert organic or inorganic residue.

15. Process according to any one of the preceding claims, wherein said fluorocarboxylic acid or fluorocarboxylic acid salt is completely soluble in the reaction medium.

16. Process according to any one of the preceding claims, wherein said acid salt is an alkali metal salt selected from among sodium, potassium, rubidium, caesium and francium, or a quaternary ammonium salt.

17. Process according to any one of the preceding claims, wherein the solvent is selected from among the N-disubstituted amides, including the tetrasubstituted ureas and monosubstituted lactams, and cyclic or noncyclic ethers.

18. Process according to any one of the preceding claims, wherein said compound having an electrophilic function is selected from among the halogenated or pseudohalogenated derivatives of organic sulphur compounds such as sulphenyl, sulphinyl or sulphonyl halides; sulphur oxides, particularly sulphur dioxides; the thiocyanates; the disulphides; and the carbonyl compounds, of the ketone, aldehyde, acid halide or activated ester type.

19. Process according to any one of the preceding claims, wherein said compound having at least one electrophilic function does not comprise a hydrogen which can be removed by a strong base.

20. Process according to any one of the preceding claims, wherein the microwave power applied is at least 1 Watt per second and per kg of reaction mass.

21. Process according to any one of the preceding claims, wherein the microwave power applied is at least 5 Watts per second and per mol of fluorocarboxylic acid.

22. Process according to any one of the preceding claims, wherein the microwave power applied is at least 100 Watts per second and per kg of reaction mass.

23. Process according to any one of the preceding claims, wherein the temperature of the reaction medium at the exit from the microwave exposure zone is maintained at not more than 150°C.

24. Process according to any one of the preceding claims, wherein said compound having an electrophilic function is sulphur dioxide.

25. Process according to claim 24, wherein the reaction medium in step a) is a liquid in equilibrium with a gaseous phase containing sulphur dioxide.

26. Process according to claim 24 or 25, which further comprises a step c) of oxidising the sulphinic acid salt obtained in step b) by bringing the product of step b) into contact with an oxidising reagent.

27. process for grafting a substituted difluoromethyl group onto a compound having at least one electrophilic function comprising the steps of:
i) bringing said compound having at least one electrophilic function into contact with a nucleophilic reagent comprising a fluorocarboxylic acid of formula Ea-CF₂-COOH where Ea denotes an electron-attracting atom or group, at least partially salified by an organic or inorganic cation, said compound having at least one electrophilic function acting as both solvent and reaction substrate; and
ii) exposing the reaction mixture to the action of microwaves.

28. Use of a process according to any one of the preceding claims in the preparation of sulphoxylated and fluorinated organic derivatives.
